# FASCICULE DE BREVET EUROPEEN

(11) **EP 1 333 812 B1**
(45) Date de publication et mention de la délivrance du brevet: **27.02.2008**
(21) Numéro de dépôt: 01996363.6
(22) Date de dépôt: 15.11.2001
(51) Int. Cl.: A61K 9/16, A61K 9/50

(54) **MICROGRANULES A BASE DE PRINCIPE ACTIF ET LEUR PROCEDE DE FABRICATION**
MIKROGRANULAT AUF WIRKSTOFFBASIS UND VERFAHREN ZU SEINER HERSTELLUNG
MICROGRANULES BASED ON ACTIVE PRINCIPLE AND METHOD FOR MAKING SAME

(30) Priorité: 16.11.2000 FR 0014803
(43) Date de publication de la demande: 13.08.2003
(73) Titulaire: ETHYPHARM, 78550 Houdan (FR)
(72) Inventeur: BRUNA, Etienne, F-28300 JOUY (FR); GENDROT, Edouard, F-28500 GARNAY (FR); COUSIN, Gérard, 28210 VILLEMEUX (FR)
(74) Mandataire: Vuillermoz, Bruno
(86) Numéro de dépôt international: PCT/FR2001/003584
(87) Numéro de publication internationale: WO 2002/039981

(56) Documents cités:
- US-A- 4 957 746
- US-A- 5 476 667

## Description

L'invention concerne un microgranule constitué d'un noyau enrobé comprenant au moins un principe actif. Elle se rapporte également au procédé de fabrication dudit microgranule de même qu'aux compositions pharmaceutiques intégrant une pluralité dudit microgranule.

Le document WO 95/22319 décrit un procédé de fabrication par extrusion/sphéronisation de fines particules à base de principe actif. Les fines particules obtenue ont une taille comprise entre 50 µm et 1 mm. En particulier, les exemples décrivent des particules de taille égale à 300 µm comprenant jusqu'à 72% de principe actif en présence d'au moins trois excipients dont notamment un agent d'extrusion. En outre, ces mêmes exemples et notamment la formulation 1 c montre que ce procédé ne permet pas d'obtenir de fines particules de cet ordre de taille avec une concentration en principe actif élevée, égale à 95%. Enfin, les fines particules obtenues à l'issue du procédé présentent une surface trop irrégulière pour permettre un traitement ultérieur satisfaisant, l'enrobage visant à masquer le goût du principe actif nécessitant, par exemple, un taux élevé d'enrobage.

Le document EP-A-443572 décrit une composition d'enrobage susceptible d'être appliquée sur différentes formes et en particulier des microgranules désignés ici par l'expression «fine granules ». Il est indiqué qu'au moins 75% de la population de microgranules présente une taille comprise entre 1 et 500 µm. Aucune information n'est donnée concernant le concentration en principe actif au sein du microgranule.

Le document FR-A-2 419 722 décrit des microgranules de principe actif et notamment de ferritine ainsi que leur procédé de fabrication. Ces microgranules sont constitués d'un noyau comportant un premier enrobage obtenu par pulvérisation d'une suspension aqueuse de principe actif, la cohésion dudit premier enrobage avec le noyau étant assurée par dispersion uniforme, entre chaque étape de pulvérisation, de petites quantités de talc (voir notamment page 7, exemple 3). Le noyau ainsi enrobé présente en outre un second enrobage, dont la nature est fonction des caractéristiques de libération du principe actif souhaité. En pratique, le noyau proprement dit peut présenter deux formes différentes. Ainsi, dans une première forme de réalisation, le noyau est exclusivement constitué de matière inerte, par exemple du type saccharose. Dans une seconde forme de réalisation (exemple 3), le noyau se présente sous forme d'un granule à base d'agent liant (par exemple amidon) et de principe actif dans des proportions de 50/50.

Le procédé de fabrication et le microgranule ainsi obtenu présentent un certain nombre d'inconvénients. S'agissant tout d'abord du procédé de fabrication, celui-ci nécessite au moins quatre étapes, qui sont la fabrication du noyau, puis, alternativement, l'application du premier enrobage, et de la dispersion de talc et enfin l'application du second enrobage. Un tel procédé est particulièrement long et ne peut être conduit en continu. En outre, la cohésion du premier enrobage sur le noyau n'est pas toujours homogène conduisant à des noyaux enrobés présentant une surface irrégulière et augmentant de ce fait la quantité de matière nécessaire au second enrobage. S'agissant du microgranule en tant que tel, il est indiqué que le noyau avant enrobage présente une taille comprise entre 0,3 et 0,5 millimètres (voir exemples) pour une concentration en principe actif représentant seulement 50% de la masse du noyau.

Dès lors, le premier problème que se propose de résoudre l'invention est de fournir des microgranules dont le noyau, avant enrobage de la ou des couches fonctionnelles conférant au microgranule les caractéristiques de libération du principe actif et/ou de masquage de goût souhaitées, soit le plus concentré possible en principe actif.

Le second problème que se propose de résoudre l'invention est de fournir un microgranule, dont le noyau, avant enrobage de la ou des couches fonctionnelles, soit sensiblement sphérique de manière à diminuer sa surface spécifique et ainsi réduire la quantité de matière nécessaire à l'enrobage ultérieur.

Le troisième problème que se propose de résoudre l'invention est de fournir un microgranule, dont le noyau, avant enrobage de la ou des couches fonctionnelles, présente une taille la plus faible possible, avantageusement une taille médiane inférieure à 500 µm.

Le document US-A-5 476 667 décrit un procédé de fabrication de pellet contenant plus de 80 % en poids de principe actif. Le procédé mis en oeuvre consistant à granuler le principe actif avec un agent liant puis à adsorber sur le mélange fondu, obtenu de nouveau du principe actif, permet d'obtenir des microgranules de taille supérieure à 300 µm, en pratique de l'ordre de 500 µm.

L'invention a donc pour objet un microgranule constitué d'un noyau enrobé d'au moins une couche d'enrobage, ledit noyau enrobé comprenant au moins un principe actif

Ce microgranule se caractérise en ce que le noyau et ladite couche d'enrobage contiennent chacun entre 80 et 95%, en poids de principe actif, le complément à 100% étant constitué par au moins un agent liant, et en ce que le noyau enrobé présente une forme sensiblement sphérique.

En dessous d'une concentration de 80 % en principe actif, le titre du microgranule n'est pas suffisant et la proportion d'agent liant est trop importante, conduisant à augmenter la taille du microgranule. Pour une concentration supérieure à 95 %, la cohésion entre les particules de principe actif n'est pas satisfaisante du fait de la trop faible proportion d'agent liant.

Dans un mode de réalisation préféré, le noyau et la couche d'enrobage contiennent chacun entre 85 et 93 %, avantageusement 90 % en poids de principe actif

Dans une forme de réalisation avantageuse, le complément à 100 % en poids du noyau et de la couche d'enrobage est constitué exclusivement d'un agent liant.

Le choix de l'agent « liant » sera déterminé en fonction non seulement de sa capacité à lier les particules de principe actif entre elles au sein du noyau enrobé mais également des caractéristiques fonctionnelles du noyau enrobé souhaitées, que ce soit en présence ou en l'absence d'enrobage fonctionnel ultérieur. Par l'expression « caractéristiques fonctionnelles », on désigne notamment mais de façon non limitative, les propriétés de masquage de goût et/ou de libération (modifiée ou non) du principe actif.

Ces caractéristiques dépendent :
- d'une part, des caractéristiques physico-chimiques de l'agent liant utilisé (solubilité, perméabilité, température de transition vitreuse, ...) ;
- et d'autre part, de la nature du principe actif (solubilité, amertume, ...).

En d'autres termes, avant tout enrobage ultérieur fonctionnel, le microgranule de l'invention présente déjà des caractéristiques spécifiques permettant ainsi de réduire l'épaisseur de l'enrobage ultérieur et donc la taille du microgranule final.

En pratique, l'agent liant est choisi dans le groupe comprenant l'éthyl cellulose, l'hydroxypropylcellulose (HPC), la carboxyméthyl cellulose (CMC), l'hydroxypropylméthylcellulose (HPMC), les polymères acryliques, les polymères méthacryliques, le copolymère amonio-méthacrylate, le polyacrylate, le copolymère acide métacrylique et la polyvinylpyrrolidone.

Selon une autre caractéristique, l'agent liant contenu dans la couche d'enrobage et celui contenu dans le noyau peuvent être identiques ou différents.

Comme déjà dit, un autre objectif de l'invention est de réduire la taille du microgranule enrobé avant enrobage de la couche fonctionnelle ultérieure éventuelle. Le titre élevé des microgranules de l'invention permet de remplir partiellement cet objectif. Pour réduire davantage encore la taille des microgranules, la taille des particules de principe actif varie entre 10 et 30 µm.

En pratique, la taille des microgranules enrobés de l'invention avant enrobage ultérieur éventuel est inférieure à 500 µm, avantageusement comprise entre 200 et 300 µm

Bien entendu et comme déjà dit, le noyau enrobé constitutif du microgranule de l'invention peut comporter un enrobage fonctionnel supplémentaire, en général d'origine polymérique, dont la nature sera fonction des caractéristiques souhaitées de la formulation finale et notamment de masquage de goût et/ou de libération modifiée ou non de principe actif.

Du fait du procédé spécifique d'obtention qui sera décrit par la suite, les microgranules de l'invention ont en outre l'avantage d'être sensiblement sphériques, présentant ainsi une surface spécifique réduite permettant d'enrober une couche ultérieure de matière première dans des quantité réduites par rapport aux microgranules connus de l'art antérieur. Cette couche ultérieure comprend préférentiellement un polymère dont l'application sur les microgranules permettra d'atteindre les caractéristiques finales souhaitées.

Par ailleurs, les microgranules de l'invention peuvent être utilisés sous différentes formes galéniques tels que notamment sachets, gélules, suspensions liquides, suspensions sèches destinées à une reconstitution extemporanée. Ils peuvent également entrer dans la composition de comprimés orodispersibles ou non. A ce titre la taille réduite des microgranules de l'invention permet de diminuer la proportion d'excipients de compression (par exemple les diluants) nécessaire à l'obtention d'un mélange homogène avant compression, ce qui permet d'avoir une forme finale de taille et de poids plus faibles au regard des comprimés connus du même type et de diminuer également les forces de compression.

A ce titre, l'invention concerne plus particulièrement des comprimés du type multiparticulaires à délitement rapide tels que ceux décrits par le Demandeur dans le document FR-A-2 679 451, et du type à hydrodispersibilité rapide (fast dispersible) comprenant les microgranules précédemment décrits.

L'invention a également pour objet le procédé de fabrication des microgranules précédemment décrit selon lequel :
◆ dans une première étape, on pulvérise sur les particules individualisées de principe actif maintenues en suspension dans un lit d'air fluidisé, une solution de granulation comprenant au moins un agent liant dans un solvant jusqu'à obtention d'un noyau ;
◆ puis, dans une seconde étape, on enrobe le noyau formé par pulvérisation d'une suspension ou solution d'enrobage à base de particules de principe actif et d'agent liant, le noyau enrobé obtenu présentant alors une forme sensiblement sphérique.

Dans une forme de réalisation avantageuse, on intercale entre la première et la seconde étape une étape de séchage des noyaux obtenus.

Selon une autre caractéristique, le procédé peut être conduit en continu ou discontinu.

Bien entendu, le solvant dans lequel est dissous l'agent liant sera déterminé en fonction de la nature même de l'agent liant et sera choisi parmi les solvants aqueux ou organiques, seuls ou en combinaison.

Pour résoudre le problème posé d'obtenir des noyaux enrobés dont la taille est la plus faible possible en tout cas inférieure à 500 micromètres, de préférence inférieure à 350 micromètres, la taille des particules de principe actif mise en oeuvre dans la première étape est comprise entre 10 et 30 micromètres, avantageusement 25 micromètres, tandis que la taille des particules de principe actif mises en oeuvre dans la seconde étape est comprise entre 10 et 20 micromètres, avantageusement inférieure à 15 micromètres.

Bien entendu, de telles tailles de particules de principe actif peuvent être obtenues par tous procédés connus de l'homme du métier, notamment micronisation ou broyage.

Dans une forme de réalisation avantageuse, la taille des particules de principe actif mises en oeuvre dans la première étape est identique à la taille de celles mises en oeuvre dans la seconde étape.

Pour contrôler au cours du procédé de fabrication la taille des microgranules en fonction du titre en principe actif, le rapport principe actif/agent liant est constant durant les première et seconde étapes, avantageusement égal à 90/10. En conséquence, la seconde étape pourra être stoppée dès que la taille souhaitée du microgranule, inférieure à 500 µm, sera atteinte.

Selon une autre caractéristique, dans une troisième étape, on pulvérise au moins une solution d'enrobage supplémentaire, dont la composition est choisie en fonction des caractéristiques de masquage de goût et /ou de libération de principe actif souhaitée.

Comme déjà dit, le procédé de l'invention est conduit en lit d'air fluidisé, avantageusement par technique bottom spray. Les paramètres du lit fluidisé (pression, débit de pulvérisation, etc...) ne présentent pas de caractéristiques particulières et seront ajustés de manière usuelle par l'homme du métier.

L'invention et les avantages qui en découlent ressortiront mieux des exemples de réalisation suivants donnés à titre illustratif.

### Exemple 1 : Fabrication de microgranules d'ibuprofène

### a) Composition du noyau enrobé

- Ibuprofène 1 600 g
- HPMC 606* 160 g
* fabriqué par SHIN-ETSU

### b) Préparation des solutions de granulation et suspension d'enrobage

### - Solution de granulation

On introduit 40 g d'HPMC 606 dans 360 g d'eau purifiée en agitant jusqu'à dissolution complète de l'hydroxyméthylpropylcellulose.

### - Suspension d'enrobage

On mélange ensemble 1 200 g d'ibuprofène micronisé (25 µm) et 120 g d'HPMC 606 dans 3 080 g d'eau purifiée toujours en agitant jusqu'à dissolution complète de l'hydroxypropylméthylcellulose.

### c) Fabrication du noyau enrobé

On introduit 400 g d'ibuprofène de granulométrie égale à 25 micromètres dans un appareil à lit fluidisé du type GLATT GPCG 1 équipé d'une cuve Bottom Spray en maintenant le principe actif à une température suffisante pour éviter le collage tout en maintenant la masse humide.

On pulvérise ensuite la solution de granulation préparée précédemment jusqu'à obtention d'un noyau présentant une granulométrie médiane d'environ 100 micromètres.

Après séchage du noyau ainsi formé, on pulvérise la suspension d'enrobage à base de principe actif en continu jusqu'à obtention d'un granulé présentant une granulométrie médiane comprise entre 250 et 300 micromètres.

### d) Enrobage fonctionnel

On applique une dispersion polymère d'éthyl cellulose et HPMC et syloïd sur le noyau enrobé afin de masquer le goût du principe actif

### EXEMPLE 2 : Fabrication de microgranules de tinidazole

### a) Composition du noyau enrobé

- Tinidazole 1 600 g
- Eudragit ® E 100 160 g

### b) Fabrication du noyau enrobé

On répète l'exemple 1 en remplaçant l'HPMC par de l'Eudragit ® E 100 et l'eau purifiée par de l'Ethanol

L'Eudragit ® est choisi comme agent liant, mais aussi pour sa fonction d'agent masquant le goût du principe actif, tout en permettant une libération immédiate de celui-ci. Ceci permet dès lors d'améliorer le masquage de goût dès l'étape de granulation, avant l'étape éventuelle d'enrobage fonctionnel.

### EXEMPLE 3 : Fabrication de microgranules de doxycycline

### a) Composition du noyau enrobé

- doxycycline 15 kg
- PVP K90 1,5 kg

### b) Préparation des solutions de granulation et suspension d'enrobage

### - Solution de granulation

On préparation une solution de granulation de PVP K90 à 5 % (p/p) dans l'éthanol.

### - Suspension d'enrobage

On prélève 25 kg de la solution de PVP K90 à 5 % dans l'éthanol précédemment obtenu à laquelle on ajoute 10 kg de doxycycline (10 µm) dans 23,75 kg d'éthanol.

### c) Fabrication du noyau enrobé

On introduit 5 kg de doxycycline (10 µm) dans un appareil à lit d'air fluidisé du type GLATT GPCG5 équipé d'une cuve bottom spray et d'une buse 12".

On pulvérise ensuite la solution de granulation précédemment obtenue. Après séchage du noyau ainsi formé, on pulvérise la suspension d'enrobage à base de principe actif en continu jusqu'à obtention d'un granulé présentant une granulométrie médiane d'environ 257 µm.

### d) Enrobage fonctionnel

On pulvérise sur les noyaux enrobés une solution polymérique d'Eudragit ® E100 (fabriqué par Röhm) à 12,5 % (p/p) dans l'éthanol. On applique l'équivalent de 10 % (p/p calculé en polymère sec) de la masse des noyaux enrobés pour le masquage de goût.

L'invention et les avantages qui en découlent ressortent bien de la description.

On notera notamment la possibilité de fabriquer des microgranules enrobés dont le noyau enrobé présente une taille très faible, inférieure à 300 micromètres, facilitant l'enrobage fonctionnel et la mise en forme ultérieurs.

## Revendications

1. Microgranule constitué d'un noyau enrobé d'au moins une couche d'enrobage, ledit noyau enrobé comprenant au moins un principe actif, **caractérisé en ce que** le noyau et ladite couche d'enrobage contiennent chacun entre 80 et 95% en poids de principe actif, le complément à 100 % étant constitué par au moins un agent liant, et **en ce que** le noyau enrobé présente une forme sensiblement sphérique, dont la taille est inférieure à 300 µm.

2. Microgranule selon la revendication 1, **caractérisé en ce que** le complément à 100% en poids du noyau et de la couche d'enrobage constitué exclusivement d'un agent liant.

3. Microgranule selon la revendication 1, **caractérisé en ce que** l'agent liant contenu dans le noyau et l'agent liant contenu dans ladite couche d'enrobage sont identiques.

4. Microgranule selon la revendication 1, **caractérisé en ce que** l'agent liant contenu dans le noyau et l'agent liant contenu dans ladite couche d'enrobage sont différents.

5. Microgranule selon la revendication 1, **caractérisé en ce que** l'agent liant est choisi dans le groupe comprenant l'éthyl cellulose, l'hydroxypropylcellulose (HPC), la carboxyméthylcellulose (CMC), l'hydroxypropylméthylcellulose (HPMC), les polymères acryliques, les polymères méthacryliques, le copolymère amonio-méthacrylate, le polyacrylate, le copolymère acide métacrylique et la polyvinylpyrrolidone.

6. Microgranule selon la revendication 1, **caractérisée en ce que** la taille des particules de principe actif varie entre 10 et 30 µm.

7. Microgranule selon la revendication 1, **caractérisé en ce que** le noyau enrobé comporte une couche fonctionnelle supplémentaire, dont la composition est choisie en fonction des caractéristiques de masquage de goût et /ou de libération de principe actif souhaitée.

8. Comprimé multiparticulaire à délitement rapide comprenant des microgranules objets de la revendication 1.

9. Comprimé à hydrodispersibilité rapide comprenant des microgranules objets de la revendication 1.

10. Suspension sous forme sèche ou liquide comprenant les microgranules objet de la revendication 1.

11. Gélule comprenant les microgranules objet de la revendication 1.

12. Procédé de fabrication du microgranule objet de la revendication 1, selon lequel:
◆ dans une première étape, on pulvérise sur les particules individualisées de principe actif maintenues en suspension dans un lit d'air fluidisé, une solution de granulation comprenant au moins un agent liant dans un solvant jusqu'à obtention d'un noyau ;
◆ puis, dans une seconde étape, on enrobe le noyau formé par pulvérisation d'une suspension ou solution d'enrobage à base de particules de principe actif et d'agent liant, le noyau enrobé obtenu présentant alors une forme sensiblement sphérique.

13. Procédé selon la revendication 13, **caractérisé en ce qu'**on intercale entre la première étape et la seconde étape, une étape de séchage des noyaux obtenus.

14. Procédé selon la revendication 13, **caractérisé en ce que** la taille des particules de principe actif mises en oeuvre dans la première étape est comprise entre 10 et 30 micromètres, avantageusement 25 micromètres tandis que la taille des particules de principe actif mises en oeuvre dans la seconde étape est comprise entre 10 et 20 micromètres, avantageusement inférieure à 15 micromètres.

15. Procédé selon la revendication 13, **caractérisé en ce que** la taille des particules de principe actif mises en oeuvre dans la première étape est identique à celle des particules mises en oeuvre dans la seconde étape.

16. Procédé selon la revendication 13, **caractérisé en ce que** le rapport principe actif/agent liant est constant tout au long des première et seconde étapes.

17. Procédé selon la revendication 13, **caractérisé en ce que** la seconde étape est conduite jusqu'à obtention de la taille souhaitée du microgranule enrobé.

18. Procédé selon la revendication 13, **caractérisé en ce que** dans une troisième étape, on pulvérise au moins une solution d'enrobage supplémentaire, dont la composition est choisie en fonction des caractéristiques de masquage de goût et /ou de libération de principe actif souhaitée.

## Claims

1. A microgranule consisting of a core coated with at least one coating layer, said coated core comprising at least one active principle, **characterized in that** the core and said coating layer each contain between 80 and 95% by weight of active principle, the balance for 100% consisting of at least one binder, and **in that** the coated core has a substantially spherical shape, whose median size is less than 300 µm.

2. The microgranule as claimed in claim 1, **characterized in that** the balance for 100% by weight of the core and of the coating layer consisting exclusively of binder.

3. The microgranule as claimed in claim 1, **characterized in that** the binder contained in the core and the binder contained in said coating layer are identical.

4. The microgranule as claimed in claim 1, **characterized in that** the binder contained in the core and the binder contained in said coating layer are different.

5. The microgranule as claimed in claim 1, **characterized in that** the binder is chosen from the group comprising ethyl cellulose, hydroxypropyl cellulose (HPC), carboxymethyl cellulose (CMC), hydroxypropyl methyl cellulose (HPMC), acrylic polymers, methacrylic polymers, amonio-methacrylate copolymer, polyacrylate, methacrylic acid copolymer and polyvinylpyrrolidone.

6. The microgranule as claimed in claim 1, **characterized in that** the size of the particles of active principle varies between 10 and 30 µm.

7. The microgranule as claimed in claim 1, **characterized in that** the coated core comprises an additional functional layer whose composition is chosen according to the characteristics of masking of taste and/or of release of active principle desired.

8. A fast disintegrating multiparticulate tablet comprising microgranules which are the subject of claim 1.

9. A fast dispersible tablet comprising microgranules which are the subject of claim 1.

10. A suspension in dry or liquid form comprising the microgranules which are the subject of claim 1.

11. A gelatin capsule comprising the microgranules which are the subject of claim 1.

12. A method for making the microgranule which is the subject of claim 1, according to which:
◆ in a first step, a granulation solution comprising at least one binder in a solvent is sprayed onto the individualized particles of active principle maintained in suspension in a fluidized bed until a core is obtained;
◆ and then, in a second step, the core formed is coated by spraying a coating suspension or solution based on particles of active principle and binder, the coated core obtained then having a substantially spherical shape.

13. The method as claimed in claim 13, **characterized in that** a step for drying the cores obtained is intercalated between the first step and the second step.

14. The method as claimed in claim 13, **characterized in that** the size of the particles of active principle used in the first step is between 10 and 30 micrometers, advantageously 25 micrometers while the size of the particles of active principle used in the second step is between 10 and 20 micrometers, advantageously below 15 micrometers.

15. The method as claimed in claim 13, **characterized in that** the size of the particles of active principle used in the first step is identical to that of the particles used in the second step.

16. The method as claimed in claim 13, **characterized in that** the active principle/binder ratio is constant throughout the first and second steps.

17. The method as claimed in claim 13, **characterized in that** the second step is performed until the desired size of the coated microgranule is obtained.

18. The method as claimed in claim 13, **characterized in that** in a third step, at least one additional coating solution is sprayed whose composition is chosen as a function of the characteristics of masking of taste and/or of release of active principle desired.

## Patentansprüche

1. Mikrokörnchen, das aus einem Kern besteht, der mit mindestens einer Umhüllungsschicht umhüllt ist, wobei der umhüllte Kern mindestens einen Wirkstoff umfasst, **dadurch gekennzeichnet, dass** der Kern und die Umhüllungsschicht jeweils zwischen 80 und 95 Gew.-% Wirkstoff enthalten, wobei die Ergänzung auf 100% aus mindestens einem Bindemittel besteht, und dass der umhüllte Kern eine im Wesentlichen kugelige Form aufweist, dessen Größe kleiner als 300 µm ist.

2. Mikrokörnchen nach Anspruch 1, **dadurch gekennzeichnet, dass** die Ergänzung auf 100 Gew.-% des Kerns und der Umhüllungsschicht ausschließlich aus einem Bindemittel besteht.

3. Mikrokörnchen nach Anspruch 1, **dadurch gekennzeichnet, dass** das im Kern enthaltene Bindemittel und das in der Umhüllungsschicht enthaltene Bindemittel identisch sind.

4. Mikrokörnchen nach Anspruch 1, **dadurch gekennzeichnet, dass** das im Kern enthaltene Bindemittel und das in der Umhüllungsschicht enthaltene Bindemittel verschieden sind.

5. Mikrokörnchen nach Anspruch 1, **dadurch gekennzeichnet, dass** das Bindemittel aus der Gruppe ausgewählt ist, die Ethylcellulose, Hydroxypropylcellulose (HPC), Carboxymethylcellulose (CMC), Hydroxypropylmethylcellulose (HPMC), Acrylpolymere, Methacrylpolymere, Ammoniummethacrylatcopolymer, Polyacrylat, Methacrylsäurecopolymer und Polyvinylpyrrolidon umfasst.

6. Mikrokörnchen nach Anspruch 1, **dadurch gekennzeichnet, dass** die Größe der Wirkstoffpartikel zwischen 10 und 30 µm variiert.

7. Mikrokörnchen nach Anspruch 1, **dadurch gekennzeichnet, dass** der umhüllte Kern eine zusätzliche Funktionsschicht umfasst, deren Zusammensetzung in Abhängigkeit von den Merkmalen einer gewünschten Geschmacksüberdeckung und/oder Wirkstofffreisetzung abhängt.

8. Schnell zerfallende multipartikuläre Tablette, welche Mikrokörnchen umfasst, die Gegenstand des Anspruchs 1 sind.

9. Schnell in Wasser dispergierende Tablette, welche Mikrokörnchen umfasst, die Gegenstand des Anspruchs 1 sind.

10. Suspension in trockener oder flüssiger Form, welche die Mikrokörnchen umfasst, die Gegenstand des Anspruchs 1 sind.

11. Gelkapsel, welche die Mikrokörnchen umfasst, die Gegenstand des Anspruchs 1 sind.

12. Verfahren zur Herstellung von Mikrokörnchen, die Gegenstand des Anspruchs 1 sind, gemäß dem:
■ in einem ersten Schritt auf die vereinzelten Partikel des Wirkstoffs, die in einem Luftbett in Suspension gehalten werden, eine Granulierungslösung aufgestäubt wird, die mindestens ein Bindemittel in einem Lösungsmittel umfasst, bis ein Kern erhalten wird;
■ dann in einem zweiten Schritt der durch Aufstäuben einer Suspension oder einer Umhüllungslösung auf Basis von Wirkstoffpartikeln und Bindemittel ausgebildete Kern umhüllt wird, wobei der erhaltene umhüllte Kern dann eine im Wesentlichen kugelige Form aufweist.

13. Verfahren nach Anspruch 12, **dadurch gekennzeichnet, dass** zwischen den ersten Schritt und den zweiten Schritt ein Schritt zum Trocknen der erhaltenen Kerne eingeschoben wird.

14. Verfahren nach Anspruch 12, **dadurch gekennzeichnet, dass** die Größe der im ersten Schritt eingesetzten Wirkstoffpartikel zwischen 10 und 30 Mikrometer, vorteilhafter Weise 25 Mikrometer beträgt, während die Größe der im zweiten Schritt eingesetzten Wirkstoffpartikel zwischen 10 und 20 Mikrometer, vorteilhafter Weise unter 15 Mikrometer beträgt.

15. Verfahren nach Anspruch 12, **dadurch gekennzeichnet, dass** die Größe der im ersten Schritt eingesetzten Wirkstoffpartikel identisch zu derjenigen der im zweiten Schritt eingesetzten Partikel ist.

16. Verfahren nach Anspruch 12, **dadurch gekennzeichnet, dass** das Verhältnis Wirkstoff/Bindemittel während des gesamten ersten und zweiten Schritts konstant ist.

17. Verfahren nach Anspruch 12, **dadurch gekennzeichnet, dass** der zweite Schritt bis zum Erhalt der gewünschten Größe des umhüllten Mikrokörnchens durchgeführt wird.

18. Verfahren nach Anspruch 12, **dadurch gekennzeichnet, dass** in einem dritten Schritt mindestens eine zusätzliche Umhüllungslösung aufgestäubt wird, deren Zusammensetzung in Abhängigkeit der Merkmale einer gewünschten Geschmacksüberdeckung und/oder Wirkstofffreisetzung gewählt ist.
